# EUROPEAN PATENT APPLICATION

(11) **EP 4 696 246 A1**
(43) Date of publication of application: **18.02.2026**
(21) Application number: 25734178.4
(22) Date of filing: 02.07.2025
(51) Int. Cl.: A61B 10/00, A61B 5/01, A61B 5/00

(54) **ELECTRONIC DEVICE AND METHOD FOR SELECTING WEARABLE ELECTRONIC DEVICE FOR MEASURING SKIN TEMPERATURE OF USER, AND NON-TRANSITORY STORAGE MEDIUM**

(30) Priority: 05.07.2024 KR 20240089231; 22.08.2024 KR 20240112947
(71) Applicant: Samsung Electronics Co., Ltd, Suwon-si, Gyeonggi-do 16677 (KR)
(72) Inventor: KIM, Yeojin, Suwon-si, Gyeonggi-do 16677 (KR)
(74) Representative: Nederlandsch Octrooibureau
(86) International application number: PCT/KR2025/009453
(87) International publication number: WO 2026/010370

(57) **Abstract**

This document relates to an electronic device, a method, and a non-transitory storage medium for selecting a wearable electronic device for measuring skin temperature of a user. According to an embodiment, the electronic device may include a display, communication circuitry, at least one processor, and memory. The memory may store instructions that, when executed by the at least one processor individually or collectively, cause the electronic device to, execute a function for predicting a menstrual cycle of a user by using skin temperature of the user; based on identifying an execution request of a function for predicting a menstrual cycle of a user by using skin temperature, obtain configuration information related to the menstrual cycle of the user stored in the memory, based on identifying that a first wearable electronic device and a second wearable electronic device are communicatively connected to the electronic device by using the communication circuit, control the display to display a first message for selecting a skin temperature measuring device at a start time point, so as to identify the start time point and an end time point of a cycle for measuring skin temperature of the user, based on the configuration information, based on identifying that the first wearable electronic device is selected as the skin temperature measuring device among the first wearable electronic device and the second wearable electronic device, transmit, to the first wearable electronic device, a first command to cause the first wearable electronic device to measure the skin temperature of the user from the start time point to the end time point, after transmitting the first command, control the display to display a second message inducing the user to keep wearing the first wearable electronic device during a first specified period of time, and predict the menstrual cycle of the user, based on skin temperature information of the user measured by the first wearable electronic device from the start time point to the end time point. Various other embodiments are also possible.

## Description

### [Technical Field]

The disclosure relates to an electronic device, a method, and a non-transitory storage medium for selecting a wearable electronic device for measuring skin temperature of a user.

### [Background Art]

An electronic device is provided in various forms such as a smartphone, a tablet personal computer (tablet PC), or a personal digital assistant (PDA) along with the development of digital technology. An electronic device is also being developed in wearable forms to enhance portability and accessibility for a user.

As communication technology develops, a wearable electronic device is becoming miniaturized or lightweight enough to be used without significant inconvenience even when worn on a user's body. For example, a wearable electronic device, such as a head-mounted display device (HMD), a smartwatch (or band), a contact lens-type device, a ring-type device, a glove-type device, a shoe-type device, or a clothing-type device, is being commercialized. Since a wearable electronic device is worn directly on the body, portability and accessibility for a user can be improved. Recently, in line with consumer trends that emphasize design, in the development of wearable electronic devices, the external design of a wearable electronic device and the usability of the wearable electronic device are being considered as important factors.

Recently, interest in healthcare services has increased, and various functions for healthcare services are being provided. Accordingly, an electronic device is evolving into various forms to measure and utilize various biometric signals of the human body. A wearable electronic device can be used to measure skin temperature among various biometric signals, and various healthcare functions using the measured skin temperature are being provided.

The above-described information may be provided as related art for the purpose of facilitating an understanding of the disclosure. No claim or determination is made as to whether any of the foregoing is applicable as prior art with respect to the disclosure.

### [Detailed Description of the Invention]

### [Technical Solution]

An electronic device may use skin temperature measured using a wearable electronic device for a function of predicting a menstrual cycle among various functions for healthcare.

In order to predict a menstrual cycle by using skin temperature, it is necessary to identify skin temperature that gradually increases starting from a predicted ovulation date, and the skin temperature measured at each body part (e.g., a wrist and a finger) of a user may differ depending on the body part. When a plurality of wearable electronic devices are connected to the electronic device and all of the plurality of wearable electronic devices are used for measuring skin temperature, predicted information of a menstrual cycle may change frequently, and thus it may be difficult for the electronic device to predict an accurate menstrual cycle.

The disclosure is to provide an electronic device, a method, and a non-transitory storage medium for selecting one of a plurality of wearable electronic devices as a wearable electronic device for measuring skin temperature in order to predict a menstrual cycle of a user when the plurality of wearable electronic devices are connected to the electronic device.

According to an embodiment of the disclosure, the electronic device includes a display, communication circuitry, at least one processor, and memory.

According to an embodiment, the memory stores an instruction that, when individually or collectively executed by the at least one processor, causes the electronic device to, execute a function for predicting a menstrual cycle of a user by using skin temperature of the user.

According to an embodiment, the memory stores an instruction that, when individually or collectively executed by the at least one processor, causes the electronic device to, based on identifying an execution request of the function for predicting the menstrual cycle of the user by using the skin temperature, obtain configuration information related to the menstrual cycle of the user stored in the memory.

According to an embodiment, the memory stores an instruction that, when individually or collectively executed by the at least one processor, causes the electronic device to identify a start time point and an end time point of a cycle for measuring skin temperature of the user, based on the configuration information.

According to an embodiment, the memory stores an instruction that, when individually or collectively executed by the at least one processor, causes the electronic device to, based on identifying that a first wearable electronic device and a second wearable electronic device are communicatively connected to the electronic device by using the communication circuitry, control the display to display a first message for selecting a skin temperature measuring device at the start time point.

According to an embodiment, the memory stores an instruction that, when individually or collectively executed by the at least one processor, causes the electronic device to, based on identifying that the first wearable electronic device is selected as the skin temperature measuring device among the first wearable electronic device and the second wearable electronic device, transmit, to the first wearable electronic device, a first command to cause the first wearable electronic device to measure the skin temperature of the user from the start time point to the end time point.

According to an embodiment, the memory stores an instruction that, when individually or collectively executed by the at least one processor, causes the electronic device to, after transmitting the first command, control the display to display a second message inducing the user to keep wearing the first wearable electronic device during a first specified period of time.

According to an embodiment, the memory stores an instruction that, when individually or collectively executed by the at least one processor, causes the electronic device to predict the menstrual cycle of the user, based on skin temperature information of the user measured by the first wearable electronic device from the start time point to the end time point.

An operation method in an electronic device according to an embodiment of the disclosure includes, executing a function for predicting a menstrual cycle of a user by using skin temperature of the user.

According to an embodiment, the method includes, based on identifying an execution request of the function for predicting the menstrual cycle of the user by using the skin temperature, obtaining configuration information related to the menstrual cycle of the user stored in memory of the electronic device.

According to an embodiment, the method includes identifying a start time point and an end time point of a cycle for measuring skin temperature of the user, based on the configuration information.

According to an embodiment, the method includes, based on identifying that a first wearable electronic device and a second wearable electronic device are communicatively connected to the electronic device by using communication circuitry of the electronic device, controlling a display of the electronic device to display a first message for selecting a skin temperature measuring device at the start time point.

According to an embodiment, the method includes, based on identifying that the first wearable electronic device is selected as the skin temperature measuring device among the first wearable electronic device and the second wearable electronic device, transmitting, to the first wearable electronic device, a first command to cause the first wearable electronic device to measure the skin temperature of the user from the start time point to the end time point.

According to an embodiment, the method includes, after transmitting the first command, controlling the display to display a second message inducing the user to keep wearing the first wearable electronic device during a first specified period of time.

According to an embodiment, the method includes predicting the menstrual cycle of the user, based on skin temperature information of the user measured by the first wearable electronic device from the start time point to the end time point.

According to an embodiment, in a non-transitory storage medium storing one or more programs, the programs include an instruction that, when executed by at least one processor of an electronic device, causes the electronic device to execute, execute a function for predicting a menstrual cycle of a user by using skin temperature of the user.

According to an embodiment, the programs include an instruction that, when executed by the at least one processor of the electronic device, causes the electronic device to execute, based on identifying an execution request of the function for predicting the menstrual cycle of the user by using the skin temperature, obtaining configuration information related to the menstrual cycle of the user stored in memory of the electronic device.

According to an embodiment, the programs include an instruction that, when executed by the at least one processor of the electronic device, causes the electronic device to execute identifying a start time point and an end time point of a cycle for measuring skin temperature of the user, based on the configuration information.

According to an embodiment, the programs include an instruction that, when executed by the at least one processor of the electronic device, causes the electronic device to execute, based on identifying that a first wearable electronic device and a second wearable electronic device are communicatively connected to the electronic device by using communication circuitry of the electronic device, controlling a display of the electronic device to display a first message for selecting a skin temperature measuring device at the start time point.

According to an embodiment, the programs include an instruction that, when executed by the at least one processor of the electronic device, causes the electronic device to execute, based on identifying that the first wearable electronic device is selected as the skin temperature measuring device among the first wearable electronic device and the second wearable electronic device, transmitting, to the first wearable electronic device, a first command to cause the first wearable electronic device to measure the skin temperature of the user from the start time point to the end time point.

According to an embodiment, the programs include an instruction that, when executed by the at least one processor of the electronic device, causes the electronic device to execute, after transmitting the first command, controlling the display to display a second message inducing the user to keep wearing the first wearable electronic device during a first specified period of time.

According to an embodiment, the programs include an instruction that, when executed by the at least one processor of the electronic device, causes the electronic device to execute predicting the menstrual cycle of the user, based on skin temperature information of the user measured by the first wearable electronic device from the start time point to the end time point.

### [Brief Description of Drawings]

FIG. 1 is a block diagram of an electronic device in a network environment according to various embodiments.
FIG. 2 illustrates a configuration of an electronic device according to an embodiment.
FIGS. 3A, 3B, and 3C illustrate an example of a function for predicting a menstrual cycle in an electronic device according to an embodiment.
FIGS. 4A and 4B illustrate an example of a function for predicting a menstrual cycle in an electronic device according to an embodiment.
FIGS. 5A, 5B, 5C, and 5D illustrate an example of selecting a wearable electronic device for measuring skin temperature in an electronic device according to an embodiment.
FIG. 6 is a graph illustrating a cycle for measuring skin temperature in an electronic device according to an embodiment.
FIGS. 7A, 7B, and 7C illustrate an example of selecting a wearable electronic device for measuring skin temperature in an electronic device according to an embodiment.
FIG. 8 illustrates an example of selecting a wearable electronic device for measuring skin temperature in an electronic device according to an embodiment.
FIG. 9 illustrates an example of selecting a wearable electronic device for measuring skin temperature according to an embodiment.
FIG. 10 is a graph illustrating an example of predicting a menstrual cycle by using skin temperature in an electronic device according to an embodiment.
FIG. 11 illustrates an example of an operation method in an electronic device according to an embodiment.

In relation to the description of drawings, the same or similar reference numerals may be used for the same or similar components.

### [Mode for Carrying out the Invention]

Hereinafter, embodiments of the disclosure will be described in detail with reference to the drawings so that those skilled in the art to which the disclosure belongs can easily implement the embodiments. However, the disclosure may be embodied in many different forms and is not limited to the embodiments described herein. In relation to the description of drawings, the same or similar reference numerals may be used for the same or similar components. In addition, in the drawings and related descriptions, descriptions of well-known functions and configurations may be omitted for clarity and conciseness. The term "user" used in various embodiments of the disclosure may refer to a person who uses an electronic device or a device (e.g., an artificial intelligence electronic device) using the electronic device.

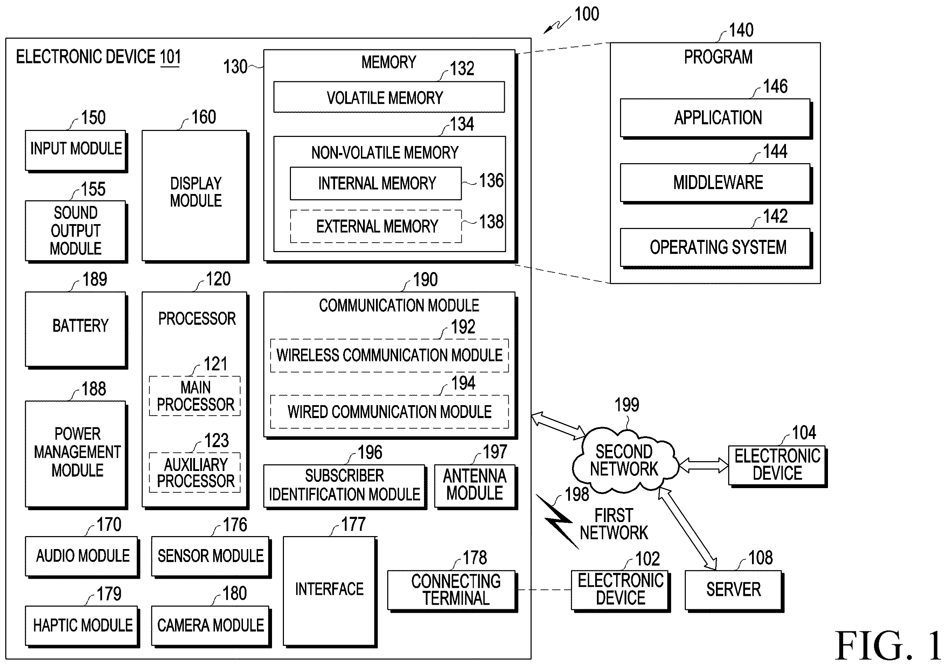

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to an embodiment, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

Hereinafter, the disclosure will specifically describe, with reference to the drawings, a configuration and an operation method of an electronic device for selecting a wearable electronic device for measuring skin temperature of a user. "Device change" described in the disclosure refers to changing (or configuring a device as a target device) a target device for obtaining skin temperature information. An electronic device may periodically obtain skin temperature information from two or more wearable devices, and in this case, "selecting a device" refers to utilizing, in prediction of a biological cycle and other functions, skin temperature information obtained from a selected wearable device. Further, in this case, "changing a device" refers to changing a selected wearable device from a first device to a second device.

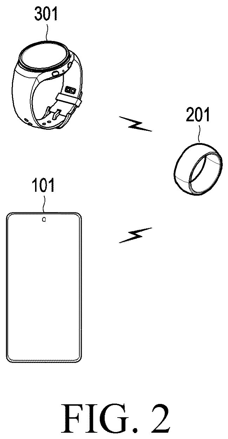

FIG. 2 illustrates a configuration of an electronic device according to an embodiment.

Referring to FIGS. 1 and 2, an electronic device 101 (e.g., the electronic device 101 of FIG. 1) according to an embodiment may collect biometric information of a user by using a plurality of wearable electronic devices (e.g., the electronic devices 102 and 104 of FIG. 1) and provide a healthcare service by using the collected biometric information. According to an embodiment, the electronic device 101 may include a display 161 (e.g., the display module 160 of FIG. 1), communication circuitry (e.g., the communication module 190 of FIG. 1), at least one processor (e.g., the processor 120 of FIG. 1), and memory (e.g., the memory 130 of FIG. 1). Without being limited to the above-described configurations, the configuration of the electronic device 101 may further include other components (e.g., the components described in FIG. 1) necessary for providing a healthcare service.

According to an embodiment, the electronic device 101 may provide a service for predicting a menstrual cycle by using body skin temperature of a user among various services (e.g., functions, operations, or methods) provided by a healthcare service.

According to an embodiment, the electronic device 101 may be connected to a first wearable electronic device 201 and a second wearable electronic device 301 which are wearable on the user's body by using a wireless communication scheme or a wired communication scheme. According to an embodiment, the electronic device 101 may collect skin temperature during a period of time specified as a cycle as biometric information of the user for providing a service (e.g., a function, an operation, or a method) for predicting a menstrual cycle by using the first wearable electronic device 201 and the second wearable electronic device 301. According to an embodiment, the electronic device 101 may identify skin temperature that gradually increases starting from a predicted ovulation date by using wearable devices to predict a menstrual cycle.

According to an embodiment, the electronic device 101 may execute an application related to a healthcare service and display, on the display 161, various information provided by a service for predicting a menstrual cycle on an execution screen of the application.

According to an embodiment, in a state in which the electronic device 101 is connected to the first wearable electronic device 201 and the second wearable electronic device 301, the electronic device may perform operations for selecting or changing one of the plurality of wearable electronic devices as a skin temperature measuring device so as to periodically or continuously measure skin temperature of the same body part.

According to an embodiment, the first wearable electronic device 201 may perform wireless communication with an electronic device (e.g., the electronic device 102 or 104 of FIG. 1) through a communication network (e.g., the first network 198 or the second network 199 of FIG. 1) with the electronic device 101. The first wearable electronic device 201 according to an embodiment may be configured to be wearable on a part (e.g., a finger) of the body. The first wearable electronic device 201 according to an embodiment may be a wearable device in the form of a ring (e.g., a smart ring or a ring-type device). The first wearable electronic device 201 is illustrated as having a ring shape that can be worn on a body part (e.g., a first body part) of the user, without being limited thereto. For example, the first wearable electronic device 201 may be implemented in the form of earphones, a smart bracelet, a smart necklace (e.g., a pendant), or a smart belt.

According to an embodiment, based on the establishment of a Bluetooth communication link between electronic devices (e.g., the electronic device 101 or the second wearable electronic device 301) to be connected, the first wearable electronic device 201 may be capable of transmitting a message between the electronic devices, and the first wearable electronic device 201 worn by the user may transmit a command corresponding to each of specific movements and gestures of the user's finger to another electronic device.

According to an embodiment, the second wearable electronic device 301 may perform wireless communication with an electronic device (e.g., the electronic device 102 or 104 of FIG. 2) through a communication network (e.g., the first network 198 or the second network 199 of FIG. 1) with the electronic device 101. The first wearable electronic device 201 according to an embodiment may be configured to be wearable on a part (e.g., a wrist) of the body. The first wearable electronic device 201 according to an embodiment may be a wearable device in the form of a watch (e.g., a smart watch). By providing guidance functions such as reminders and insight messages at the start of the menstrual cycle, users can more effectively select or change their skin temperature measuring device, thereby enhancing the accuracy of menstrual cycle predictions.

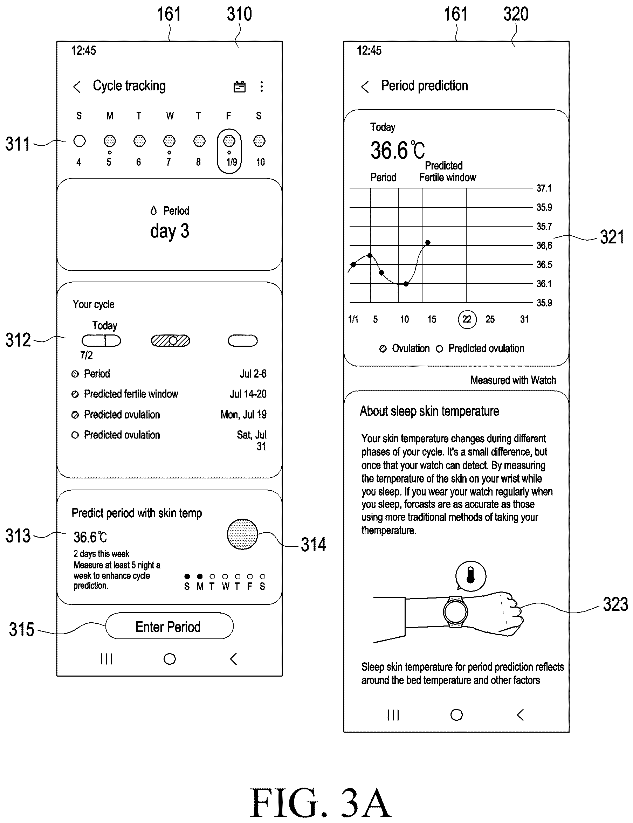

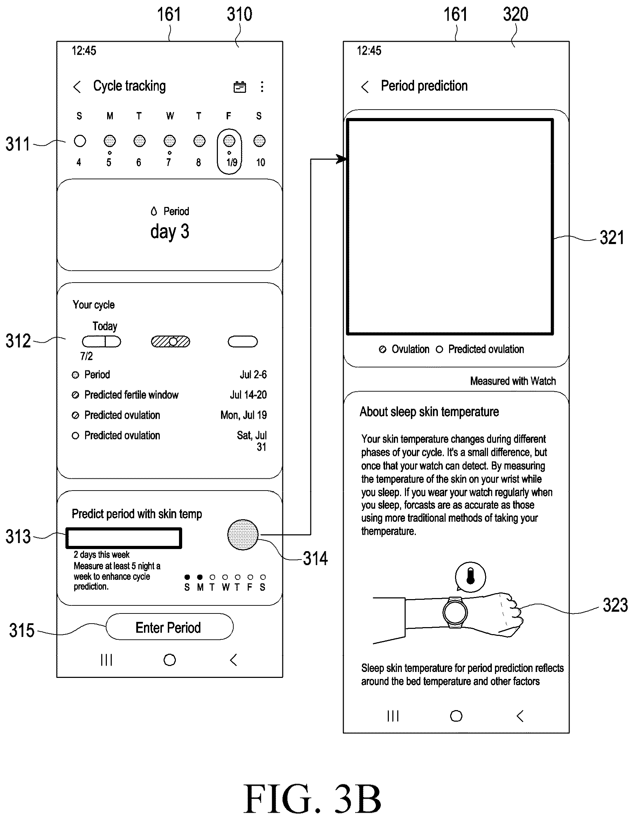

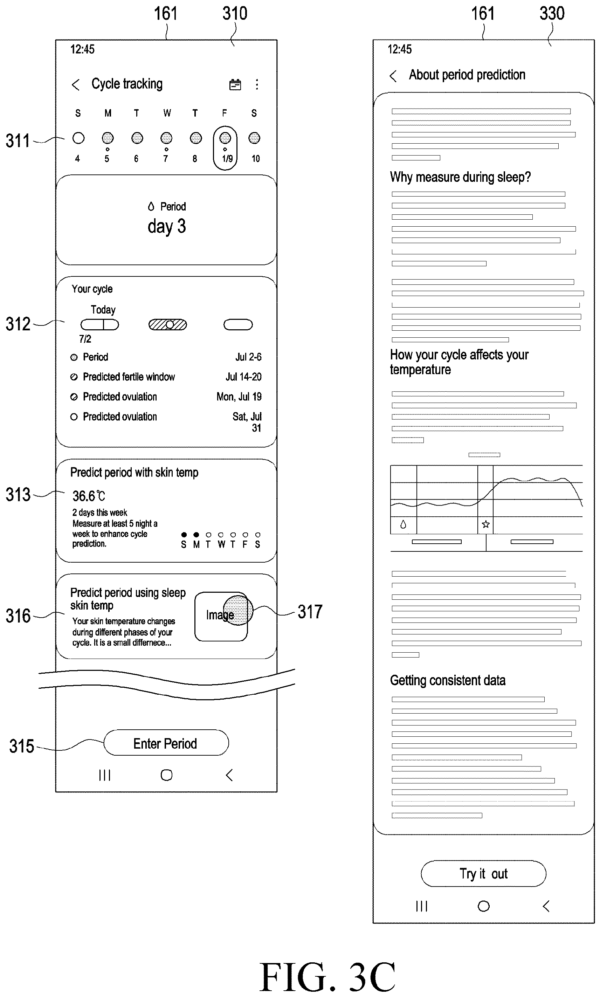

FIGS. 3A, 3B, and 3C illustrate an example of a function for predicting a menstrual cycle in an electronic device according to an embodiment.

Referring to FIGS. 1, 2, 3A, 3B, and 3C, according to an embodiment, the processor 120 of the electronic device 101 may be connected to the first wearable electronic device 201 wearable on a first body part of a user and the second wearable electronic device 301 wearable on a second body part of the user by using a wireless communication scheme or a wired communication scheme.

According to an embodiment, the processor 120 may execute an application related to a healthcare service and control the display 161 to display a first execution screen 310 (e.g., a main screen) for menstrual cycle prediction.

According to an embodiment, the electronic device 101 may collect skin temperature information obtained by measuring skin temperature of the user by using the first wearable electronic device 201 and/or the second wearable electronic device 301 to continuously identify skin temperature that gradually increases starting from a predicted ovulation date.

According to an embodiment, the processor 120 may obtain configuration information related to a menstrual cycle of the user stored in the memory 130, based on identifying an execution request of a function for menstrual cycle prediction. The configuration information may be pre-configured and stored in the memory based on user input information or information related to a menstrual cycle predicted in a previous cycle, before identifying the execution request of the function. The user input information is information directly input by the user by using an input interface of the first execution screen 310, and may include the user's gender, age, menstrual cycle, menstrual start date of a previous cycle, and/or menstrual end date of the previous cycle. The information related to the predicted menstrual cycle is information predicted (or updated) based on skin temperature information measured in the previous cycle, and may include at least one of predicted menstrual cycle, predicted menstrual start date, predicted menstrual end date, predicted ovulation date, and predicted fertile period information.

According to an embodiment, the processor 120 may configure user interfaces (e.g., screen parts) 311 and 312 of the first execution screen 310, based on the configuration information, and configure user interfaces (e.g., screen parts) 313 of the first execution screen 310, based on skin temperature information (e.g., a measured skin temperature value) collected during a specified period of time of a cycle. The processor 120 may control the display 161 to display the first execution screen 310 including the configured user interfaces (e.g., screen parts) 311, 312, and 313 and at least one function button 315 (e.g., an object, a menu, or a graphic element) (e.g., enter period). The processor 120 may control the display 161 to display a menstrual period (e.g., 3 to 5 days) (e.g., a menstrual start date and/or a menstrual end date) as a specified graphic element on a calendar provided in a first user interface 311 of the first execution screen 310, and to display a predicted fertile period and a predicted ovulation date as specified graphic elements. The processor 120 may display information (e.g., a menstrual period, a predicted fertile period, and a predicted ovulation date) related to the user's predicted menstrual cycle on a second user interface 312 of the first execution screen 310. The processor 120 may control the display 161 to display a skin temperature value measured by a selected wearable electronic device (the first wearable electronic device 201 or the second wearable electronic device 301) and information (e.g., an object or a guidance message indicating a measurement period (number of days)) related to skin temperature measurement on a third user interface 313 of the first execution screen 310.

According to an embodiment, when the processor 120 identifies that skin temperature is measured by a selected measuring device (e.g., the first wearable electronic device 201) and identifies a selection input 314 of a function button (e.g., an object, a menu, or a graphic element) displayed on the third user interface 313 of the first execution screen 310, as illustrated in FIG. 3A, the processor may configure user interfaces 321 and 323 including skin temperature information (e.g., first skin temperature information) measured during a specified period of time of a cycle, and control the display 161 to display the user interfaces 321 and 323 on a second execution screen 320. The processor 120 may control the display 161 to display an object (e.g., an image) representing the selected measuring device on the third user interface 313. For example, when the user selects the first wearable electronic device 201 (e.g., a ring), the processor 120 may control the display 161 to display an object (e.g., a ring image) representing the first wearable electronic device 201 on the third user interface 313. For example, when the user selects the second wearable electronic device 301 (e.g., a watch), the processor 120 may control the display 161 to display an object (e.g., a watch image) representing the second wearable electronic device 301 on the third user interface 313. The processor 120 may control the display 161 to display a skin temperature value measured on the current day (e.g., 36.6 degrees) and skin temperature information (e.g., a graph) measured during a specified period of time of a cycle on a first user interface 321 of the second execution screen 320. The processor 120 may control the display 161 to display guidance information on skin temperature measurement during a specified period of time (e.g., during bedtime) on a second user interface 323 of the second execution screen 320.

According to an embodiment, when the processor 120 identifies that skin temperature is measured by an unselected wearable electronic device (e.g., the second wearable electronic device 301) and identifies the selection input 314 of the function button (e.g., an object, a menu, or a graphic element) displayed on the third user interface 313 of the first execution screen 310, as illustrated in FIG. 3B, the processor may apply a graphic effect to the third user interface 313 of the first execution screen 310 and the first user interface 321 of the second execution screen 320 such that measured skin temperature information is not displayed (e.g., displayed as an empty area) or is hidden on the third user interface 313 and the first user interface 321. When skin temperature information measured by the selected wearable electronic device (e.g., the first wearable electronic device 201) is not collected (e.g., when a selected user does not wear a wearable electronic device), the processor 120 may apply a message or a graphic effect indicating that there is no measured skin temperature information on a user interface to the third user interface 313 and the first user interface 321. The second user interface 323 of FIGS. 3A and 3B illustrates, for example, a description and an image for measuring skin temperature by the second wearable electronic device (e.g., a watch), without being limited thereto. When a wearable electronic device selected for measuring skin temperature is the first wearable electronic device (e.g., a ring) 201, the processor 120 of the electronic device 101 may display, on the second user interface 323, a description and an image for measuring skin temperature by the first wearable electronic device 201.

According to an embodiment, as illustrated in FIG. 3C, the processor 120 may further configure a fourth user interface 316 including a description of a function for predicting a menstrual cycle by using skin temperature during a specified period of time (e.g., during bedtime), and control the display 161 to display the fourth user interface on the first execution screen 310. When the user selects (317) a function button (e.g., an object, a menu, or a graphic element) included in the fourth user interface 316, the processor 120 may control the display 161 to display a third execution screen 330 including a detailed description (e.g., a detailed page) of the function for predicting a menstrual cycle and a function button (e.g., a try it out button). In this way, by providing guidance functions such as reminders and insight messages at the start of the menstrual cycle, users can more effectively select or change their skin temperature measuring device, thereby enhancing the accuracy of menstrual cycle predictions.

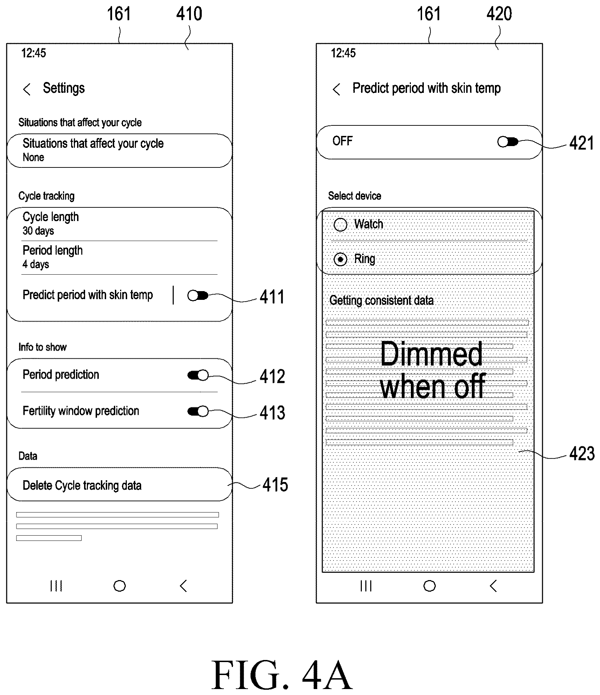

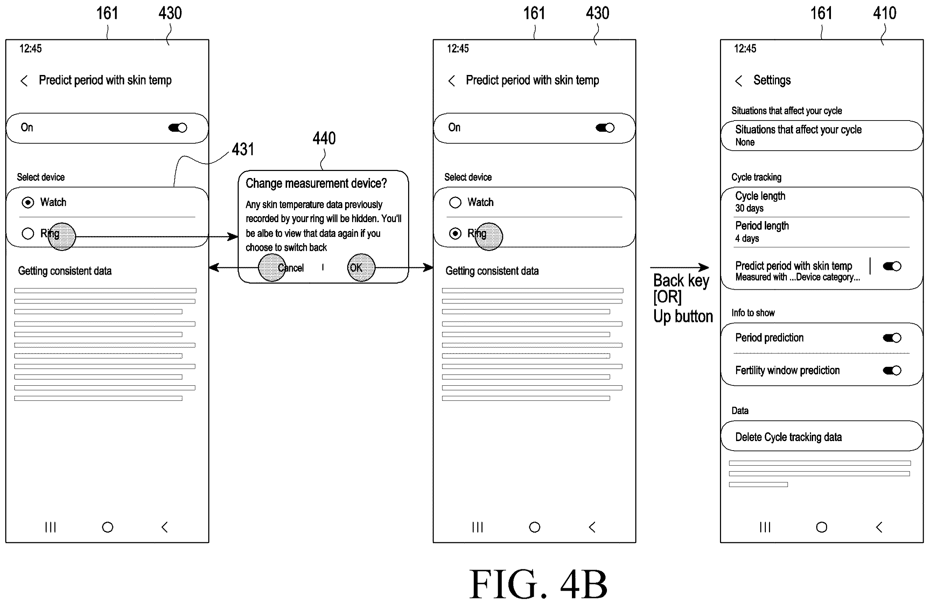

FIGS. 4A and 4B illustrate an example of a function for predicting a menstrual cycle in an electronic device according to an embodiment.

Referring to FIGS. 1, 2, 4A, and 4B, according to an embodiment, the processor 120 of the electronic device 101 may provide interfaces 411, 412, and 413 related to a function for predicting a menstrual cycle by using a configuration screen 410 of an application related to a healthcare service, before executing the function for predicting a menstrual cycle. The interface 411 may include a menu (e.g., "predict period with skin temp" and a selection button) for configuring whether to execute the function for predicting a menstrual cycle, information (e.g., cycle length 30 days) on a menstrual cycle input by a user, and information (e.g., period length 4 days) on a menstrual period. The interface 412 may provide a user interface 412 for configuring information (e.g., cycle prediction and a predicted fertile period window) shown on a first execution screen of the function for predicting a menstrual cycle. The interface 415 may include a menu and a description for deleting cycle tracking data.

According to an embodiment, as illustrated in FIG. 4A, the processor 120 may configure the function for predicting a menstrual cycle not to be executed when the user inputs a selection button included in an interface 421 to "off", and control the display 161 such that a user interface (e.g., a menu or a screen part) for selecting a device for measuring skin temperature is not displayed (e.g., dimmed) (423) on a configuration screen 420.

According to an embodiment, as illustrated in FIG. 4B, the processor 120 may configure (turn on) the function for predicting a menstrual cycle to be executed when the user inputs a selection button included in the interface 411 to "on", and control the display 161 to display a configuration screen 430 including a user interface 431 for selecting a wearable electronic device for skin temperature measurement. When a wearable electronic device to be used for skin temperature measurement is selected (e.g., a ring is selected) on the configuration screen 430, the processor 120 may control the display 161 to display a guidance message 440 for identifying whether to change to the wearable electronic device (e.g., a ring) selected as a skin temperature measuring device. When the user accepts (ok) the change to the wearable electronic device selected as the skin temperature measuring device by using the guidance message 440, the processor 120 may control the display 161 to display, on the configuration screen 430, a graphic element indicating the execution (on) of the function for predicting a menstrual cycle and a graphic element indicating the selection of the selected wearable electronic device (e.g., a ring). When the user inputs a specified key (e.g., a back key or an up button), the processor 120 may control the display 161 to display the configuration screen 410 changed to display the graphic element indicating the execution (on) of the function for predicting a menstrual cycle. In this way, by providing guidance functions such as reminders and insight messages at the start of the menstrual cycle, users can more effectively select or change their skin temperature measuring device, thereby enhancing the accuracy of menstrual cycle predictions.

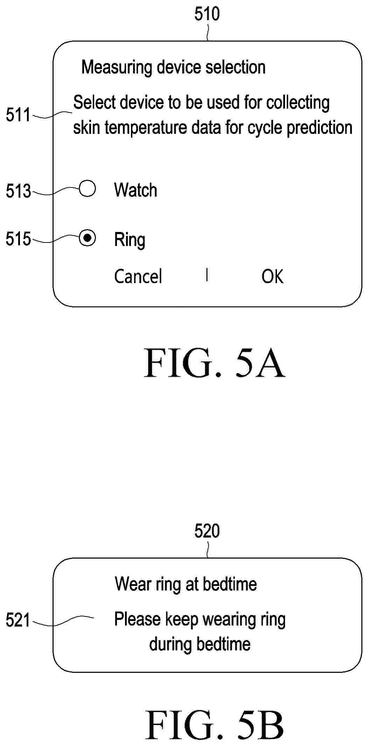

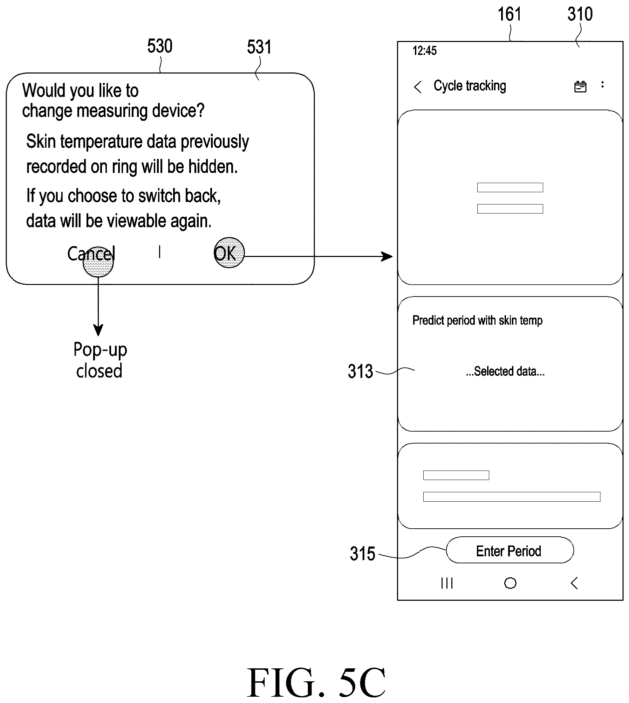

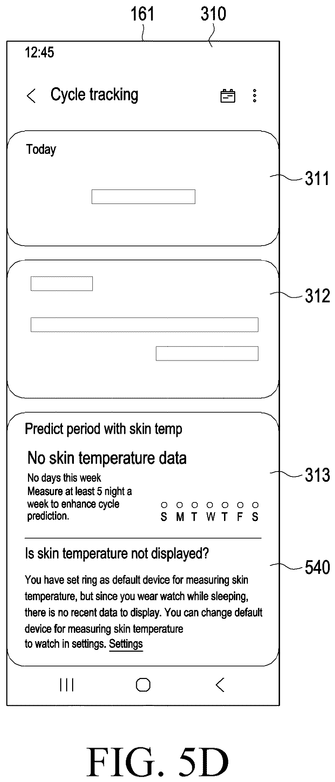

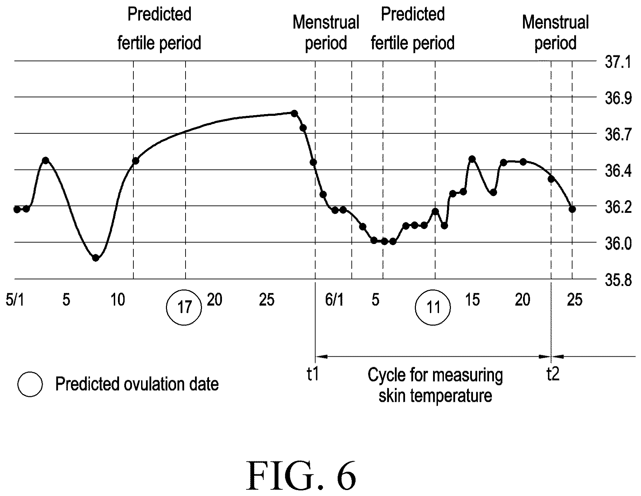

FIGS. 5A, 5B, 5C, and 5D illustrate an example of selecting a wearable electronic device for measuring skin temperature in an electronic device. FIG. 6 is a graph illustrating a cycle for measuring skin temperature in an electronic device.

Referring to FIGS. 1, 2, 3A, 3B, 5A, 5B, 5C, 5D, and 6, according to an embodiment, the processor 120 of the electronic device 101 may identify a start time point and an end time point of a cycle for measuring skin temperature of a user, based on configuration information. Here, the cycle for measuring skin temperature of the user is a cycle for measuring skin temperature necessary to predict a menstrual cycle, and may be a cycle different from the menstrual cycle. As illustrated in FIG. 6, the cycle for measuring skin temperature may be configured, for example, from a predicted menstrual start date to a predicted fertile period end date, from the predicted menstrual start date to the day before the next predicted menstrual start date, or from the predicted menstrual start date to a specified date between the predicted fertile period end date and the day before the next predicted menstrual start date. The cycle for measuring skin temperature may be configured to be the same as or different from a menstrual cycle (e.g., 30 days) included in the configuration information.

According to an embodiment, when the processor 120 identifies that the first wearable electronic device 201 and the second wearable electronic device 301 are connected to the electronic device 101 by using communication circuitry (e.g., the communication module 190 of FIG. 1), the processor may provide a message guiding (e.g., inducing or recommending) selection or change of one of the connected first wearable electronic device 201 and second wearable electronic device 301 as a skin temperature measuring device in order to predict a more accurate menstrual cycle. As illustrated in FIG. 5A, the processor 120 may control the display 161 to display a message 510 (e.g., a first message) guiding selection of a wearable electronic device for measuring skin temperature of the user at the identified start time point. The message 510 may include content 511 that guides selection of a wearable electronic device for measuring skin temperature, and function buttons (e.g., a watch selection button 513, a ring selection button 515, a cancel button, and/or an ok button). The processor 120 may control the display 161 to display the message 510 on a part of the first execution screen 310 or through a separate pop-up window adjacent to or overlapping the first execution screen 310.

According to an embodiment, when the user selects one of the watch selection button 513 and the ring selection button 515 included in the message 510, the processor 120 may transmit a command for measuring skin temperature to a wearable electronic device indicated by the selected button by using communication circuitry. For example, the processor 120 may transmit a command to measure skin temperature of the user from the start time point to the end time point to the first wearable electronic device 201 in response to a selection input of the ring selection button 515. For example, the processor 120 may transmit a command to measure skin temperature of the user from the start time point to the end time point to the second wearable electronic device 301 in response to a selection input of the watch selection button 513. Without being limited thereto, the processor 120 according to an embodiment may be configured to allow selection of both the watch selection button 513 and the ring selection button 515 included in the message 510, and when the user selects both the watch selection button 513 and the ring selection button 515, the processor may transmit a command to measure skin temperature of the user from the start time point to the end time point to both the first wearable electronic device 201 and the second wearable electronic device 301. The processor 120 according to an embodiment may obtain (e.g., obtain information from a server) analysis result information obtained by analyzing skin temperature of the user measured from the start time point to the end time point by each of the first wearable electronic device 201 and the second wearable electronic device 301, and control the display 161 to display the analysis result information for each of the first wearable electronic device 201 and the second wearable electronic device 301. The processor 120 may control the display 161 to display a message for informing the user that there may be a difference in skin temperature measured by the first wearable electronic device 201 and the second wearable electronic device 301 depending on a measurement part, based on the obtained analysis result. The processor 120 may assign a higher priority to a device having higher accuracy among the first wearable electronic device 201 and the second wearable electronic device 301, based on the analysis result information for each of the first wearable electronic device 201 and the second wearable electronic device 301 and actual menstrual start data according to an input of the user.

According to an embodiment, as illustrated in FIG. 5B, after transmitting the command, the processor 120 may control the display 161 to display a message 520 (e.g., a second message) inducing the user to keep wearing the first wearable electronic device 201 during a specified period of time (e.g., bedtime). A message 520 may include content (e.g., "Wear the ring at bedtime") that induces the user to wear the first wearable electronic device 201 (e.g., a ring) at bedtime and content (e.g., "Please keep wearing the ring during bedtime") that induces the user to keep wearing the device. The processor 120 may control the display 161 to display the message 520 on a part of the first execution screen 310 or through a separate pop-up window adjacent to or overlapping the first execution screen 310.

According to an embodiment, the processor 120 may display a message 530 (e.g., a third message) for identifying whether to change to a wearable electronic device (e.g., the first wearable electronic device 201) selected as the skin temperature measuring device. For example, the processor 120 may not display the message 530 according to a function configuration. The message 530 may include guidance content 531 on changing the skin temperature measuring device (e.g., "Would you like to change the measuring device?" and "The skin temperature data previously recorded by the watch will be hidden. If you choose to switch back, the data will be viewable again.") and function buttons (e.g., a cancel button and an ok button). The processor 120 may control the display 161 to display the message 530 on a part of the first execution screen 310 or through a separate pop-up window adjacent to or overlapping the first execution screen 310. When the user selects a button (ok button) for accepting a change of the skin temperature measuring device, the processor 120 may control the display 161 to display skin temperature information (e.g., first skin temperature information) measured by the selected wearable electronic device (e.g., the first wearable electronic device 201) on the third user interface 313 of the first execution screen 310.

According to an embodiment, the processor 120 may predict a menstrual cycle of the user, based on skin temperature information of the user measured on a first body part of the user by the first wearable electronic device 201 from a start time point to an end time point of a cycle for measuring skin temperature. For example, the processor 120 may predict a menstrual cycle of the user, based on the skin temperature information collected during the cycle at the end time point. The processor 120 may update a menstrual cycle predicted in a previous cycle to a menstrual cycle predicted in a current cycle. The processor 120 may obtain information related to the menstrual cycle predicted in the current cycle and control the display 161 to display the obtained information related to the menstrual cycle on the first execution screen 310 (e.g., reconfigure and display the first execution screen based on the obtained information related to the menstrual cycle).

According to an embodiment, as illustrated in FIG. 5D, when the processor 120 identifies that the selected wearable electronic device (e.g., the first wearable electronic device 201) is not worn during a specified period of time (e.g., 2 days) after a start time point of a cycle, the processor may control the display 161 to display a message (e.g., a fourth message, the message 520 of FIG. 5B) for inducing (reminding) wearing of the selected wearable electronic device, while displaying, on the first execution screen 310, a message 540 for identifying whether skin temperature information is not displayed and guiding that the skin temperature measuring device can be changed. The processor 120 may display the message 540 on the first execution screen 310 or through a separate pop-up window adjacent to or overlapping the first execution screen 310. In this way, by providing guidance functions such as reminders and insight messages at the start of the menstrual cycle, users can more effectively select or change their skin temperature measuring device, thereby enhancing the accuracy of menstrual cycle predictions.

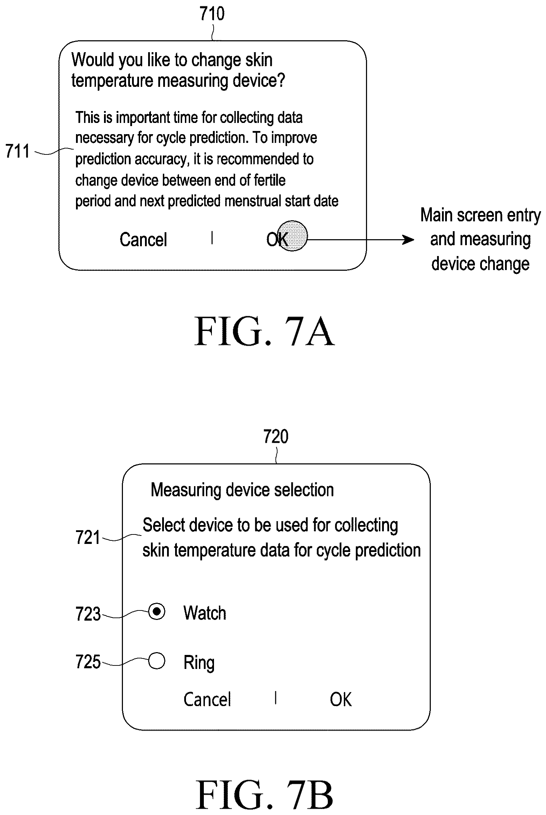

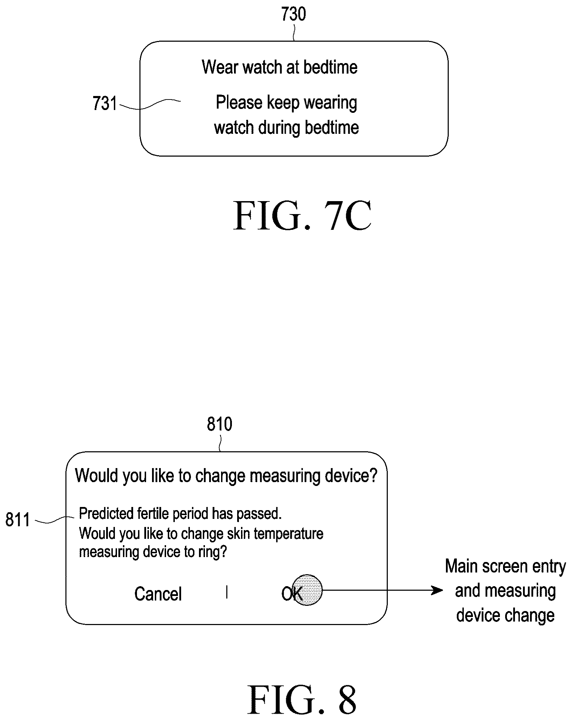

FIGS. 7A, 7B, and 7C illustrate an example of selecting a wearable electronic device for measuring skin temperature in an electronic device according to an embodiment.

Referring to FIGS. 1, 2, 7A, 7B, and 7C, according to an embodiment, when the processor 120 of the electronic device 101 identifies a request for changing a skin temperature measuring device before an end time point of a cycle for measuring skin temperature (e.g., just before a predicted fertile period or at the beginning of a menstrual cycle), which is a non-recommended time, the processor may control a display to display a message 710 (e.g., a fifth message) indicating the importance of menstrual cycle prediction and recommending changing of the skin temperature measuring device at a start time point of the next cycle. The processor 120 may control the display 161 to display the message 710 on a part of the first execution screen 310 or through a separate pop-up window adjacent to or overlapping the first execution screen (e.g., the first execution screen 310 or a main screen of FIG. 3). For example, the message 710 may include content 711 indicating the importance of menstrual cycle prediction and recommending changing of the skin temperature measuring device at the start time point of the next cycle (e.g., "Would you like to change the skin temperature measuring device?" and "This is an important time for collecting data necessary for cycle prediction. To improve prediction accuracy, it is recommended to change the device between a predicted fertile period end date and the next predicted menstrual start date.") and function buttons (e.g., a cancel button and an ok button). For example, when a user inputs an acceptance button (ok button) included in the message 710, the processor 120 may configure the skin temperature measuring device to be changed, transmit a command to measure skin temperature during a specified period of time of a cycle to a wearable electronic device (e.g., the second wearable electronic device 301) changed as the skin temperature measuring device, and control the display 161 to display the first execution screen (e.g., a main screen). According to an embodiment, when the processor 120 of the electronic device 101 identifies a request for changing the skin temperature measuring device before the predicted fertile period end date, which is a non-recommended time, the processor may control the display 161 to display a message (e.g., a sixth message) not recommending changing of the skin temperature measuring device. For example, the processor 120 may display a sixth message including content that does not recommend changing of the skin temperature measuring device (e.g., "Device change is not recommended. Measure skin temperature with the selected ring"). For example, the processor 120 may display a fifth message indicating the importance of menstrual cycle prediction and guiding the user to change the skin temperature measuring device at the start time point of the next cycle, while also displaying the sixth message including the content that does not recommend changing of the skin temperature measuring device.

According to an embodiment, as illustrated in FIG. 7B, when it is an end time point of a cycle for measuring skin temperature (e.g., after a predicted fertile period end date), the processor 120 may control the display 161 to display a message 720 (e.g., the message 510 of FIG. 5A) for identifying whether to change the skin temperature measuring device. For example, when the user inputs a button 723 (e.g., a watch) indicating the second wearable electronic device 301 in the message 720, the processor 120 may change the skin temperature measuring device from the first wearable electronic device 201 to the second wearable electronic device 301 and transmit, to the second wearable electronic device 301, a command to measure skin temperature during a specified period of time of a cycle. The message 720 may include content 721 that induces selection of a wearable electronic device for measuring skin temperature, and function buttons (e.g., a watch selection button 723, a ring selection button 725, a cancel button, and/or an ok button). The processor 120 may control the display 161 to display the message 720 on a part of the first execution screen 310 or through a separate pop-up window adjacent to or overlapping the first execution screen 310.

According to an embodiment, as illustrated in FIG. 7C, after transmitting the command, the processor 120 may control the display 161 to display a message 730 (e.g., a third message) inducing the user to keep wearing the second wearable electronic device 301 during a specified period of time (e.g., bedtime) The message 730 may include content 731 that induces wearing of the second wearable electronic device 301 (e.g., a watch) at bedtime and induces the user to keep wearing the second wearable electronic device 301 (e.g., "Wear the watch at bedtime" and "Please keep wearing the watch during bedtime"). The processor 120 may control the display 161 to display the message 730 on a part of the first execution screen 310 or through a separate pop-up window adjacent to or overlapping the first execution screen 310. In this way, by providing guidance functions such as reminders and insight messages at the start of the menstrual cycle, users can more effectively select or change their skin temperature measuring device, thereby enhancing the accuracy of menstrual cycle predictions.

FIG. 8 illustrates an example of selecting a wearable electronic device for measuring skin temperature in an electronic device according to an embodiment.

Referring to FIGS. 1, 2, and 8, according to an embodiment, when it is an end time point of a cycle for measuring skin temperature (e.g., after a predicted fertile period end date), the processor 120 of the electronic device 101 may identify whether a user has attempted to change a wearable electronic device before the end time point. When the user has attempted to change the device before the end time point but keeps the selected wearable electronic device instead of changing the device, the processor 120 may control the display 161 to display a message 810 for identifying whether to change a skin temperature measuring device. The message 810 may include content 811 for identifying whether to change the wearable electronic device for measuring skin temperature (e.g., "Would you like to change the skin temperature measuring device?" and "The predicted fertile period has passed. Would you like to change the skin temperature measuring device?") and function buttons (e.g., a cancel button and an ok button). For example, when the user inputs an acceptance button (ok button) in the message 810, the processor 120 may change the skin temperature measuring device from the first wearable electronic device 201 to the second wearable electronic device 301, transmit a command to measure skin temperature during a specified period of time of a cycle to the second wearable electronic device 301, and control the display 161 to display a first execution screen (e.g., a main screen).

According to an embodiment, when a predicted fertile period has not ended or there is no attempt to change the skin temperature measuring device just before the predicted fertile period, the processor 120 may control the display 161 to display the first execution screen (e.g., a main screen) without displaying a message for identifying whether to change the skin temperature measuring device. In this way, by providing guidance functions such as reminders and insight messages at the start of the menstrual cycle, users can more effectively select or change their skin temperature measuring device, thereby enhancing the accuracy of menstrual cycle predictions.

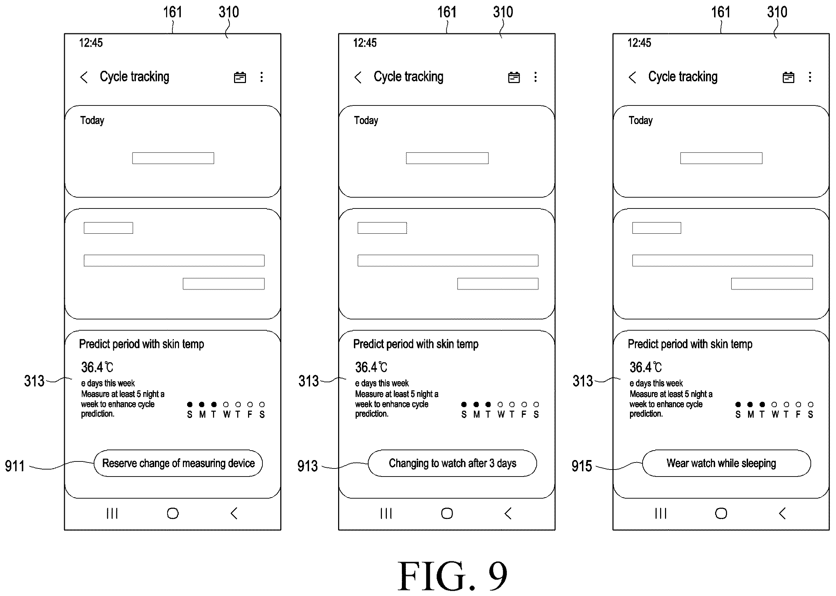

FIG. 9 illustrates an example of selecting a wearable electronic device for measuring skin temperature according to an embodiment.

Referring to FIGS. 1, 2, and 9, according to an embodiment, the processor 120 of the electronic device 101 may control the display 161 to display a function button 911 (e.g., a menu, an object, or a graphic element) for reserving a change of a skin temperature measuring device before a start time point of the next cycle on the first execution screen 310 (e.g., a main screen). The function button 911 may be displayed in an area below the third user interface 313 of the first execution screen 310 and may be displayed during a specified period of time (e.g., from 5 days before a predicted menstrual end date to a menstrual cycle end date). When a user selects the function button 911 (e.g., a "measuring device change reservation" button), the processor 120 may control the display 161 to display a message 913 (e.g., "Changing to the watch after 3 days") guiding that the skin temperature measuring device will be changed from the first wearable electronic device 201 to the second wearable electronic device 301 (e.g., a watch) before the start time point of the next menstrual cycle. The processor 120 may control the display 161 to display a message 915 (e.g., "Wear the watch while sleeping") guiding (e.g., recommending) wearing of the second wearable electronic device 301 to measure skin temperature by using the changed second wearable electronic device 301 at the start time point of the next cycle for measuring skin temperature. In this way, by providing guidance functions such as reminders and insight messages at the start of the menstrual cycle, users can more effectively select or change their skin temperature measuring device, thereby enhancing the accuracy of menstrual cycle predictions.

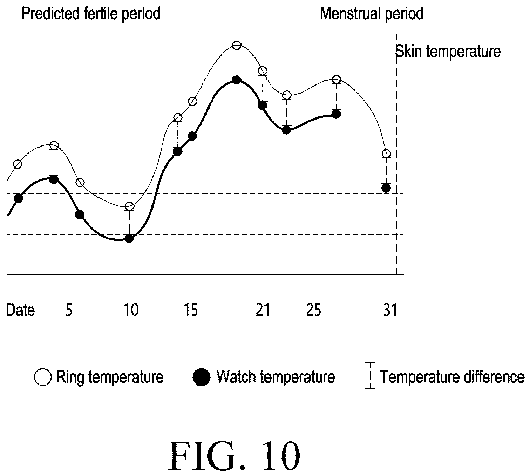

FIG. 10 is a graph illustrating an example of predicting a menstrual cycle by using skin temperature in an electronic device.

Referring to FIGS. 1, 2, and 10, the processor 120 of the electronic device 101 according to an embodiment may identify that the selected first wearable electronic device 201 is not worn during a specified period of time and the unselected second wearable electronic device 301 is worn. When the processor 120 identifies that the unselected second wearable electronic device is worn, the processor may collect second skin temperature information measured by the unselected second wearable electronic device during a specified period of time in a cycle. The processor 120 may correct the second skin temperature information by a skin temperature difference value in order to predict an accurate menstrual cycle. The skin temperature difference value may be configured (e.g., specified or calculated) as the difference between first skin temperature values measured by the first wearable electronic device and second skin temperature values measured by the second wearable electronic device during a specified period of time in a previous cycle. The first wearable electronic device 201 (e.g., a ring) may be worn on a first body part (e.g., a finger) with a higher blood vessel distribution due to the body structure, and the second wearable electronic device 301 (e.g., a watch) is worn on a body part with a relatively fewer blood vessel distribution and having more skin exposed to an external environment compared to the first body part (e.g., a finger), and thus may be relatively more affected by an external environmental temperature. The first body part on which the first wearable electronic device 201 is worn may be measured to have a higher skin temperature than that of a second body part (e.g., a wrist) on which the second wearable electronic device 301 is worn.

The electronic device 101 according to an embodiment may implement a software module (e.g., the program 140 of FIG. 1) for executing a healthcare service (e.g., a menstrual cycle prediction function).

According to an embodiment, memory (e.g., the memory 130 of FIG. 1 and the memory 270 of FIG. 4) of the electronic device may store instructions to implement the software module. At least one processor (e.g., the processor 120 of FIG. 1) may execute the instructions stored in the memory to implement the software module and control hardware (e.g., the sensor module 176, the power management module 188, or the communication module 190 of FIG. 1) associated with a function of the software module.

The software module of the electronic device according to an embodiment may be configured to include a kernel (or HAL), a framework (e.g., the middleware 144 of FIG. 1), and an application (e.g., the application 146 of FIG. 1). At least a part of the software module may be preloaded on the electronic device or may be downloadable from a server (e.g., the server 108). The application may include an application received from an external electronic device (e.g., the server 108 or the electronic device 102 or 104). According to an embodiment, the application may include a preloaded application or a third party application that is downloadable from the server. The components of the software module and names of the components according to the described embodiment may vary depending on the type of operating system. According to an embodiment, at least a part of the software module may be implemented as software, firmware, hardware, or a combination of at least two or more of these. At least a part of the software module may be implemented (e.g., executed) by, for example, a processor (e.g., an AP). At least a part of the software module may include, for example, a module, a program, a routine, a set of instructions, or a process for performing at least one function.

As such, in an embodiment, the main components of the electronic device have been described with reference to the electronic device 101 of FIGS. 1 and 2. However, in various embodiments, the components shown in FIGS. 1 and 2 are not all essential components, and the electronic device 101 may be implemented by more components than the illustrated components, or the electronic device 101 may be implemented by fewer components than the illustrated components. In addition, positions of the main components of the electronic device 101 described above with reference to FIGS. 1 and 2 can be changed according to various embodiments.

According to an embodiment, an electronic device (e.g., the electronic device 101 of FIGS. 1 and 2) may include a display (e.g., the display module 160 of FIG. 1 or the display 161 of FIGS. 3A to 5D and FIGS. 7A to 10), communication circuitry (e.g., the communication module 190 of FIG. 1), at least one processor (e.g., the processor 120 of FIG. 1), and memory (e.g., the memory 130 of FIG. 1).

According to an embodiment, the memory may store an instruction that, when individually or collectively executed by the at least one processor, causes the electronic device to execute a function for predicting a menstrual cycle of a user by using skin temperature of the user.

According to an embodiment, the memory may store an instruction that, when individually or collectively executed by the at least one processor, causes the electronic device to, based on identifying an execution request of the function for predicting the menstrual cycle of the user by using skin temperature, obtain configuration information related to the menstrual cycle of the user stored in the memory.

According to an embodiment, the memory may store an instruction that, when individually or collectively executed by the at least one processor, causes the electronic device to identify a start time point and an end time point of a cycle for measuring skin temperature of the user, based on the configuration information.

According to an embodiment, the memory may store an instruction that, when individually or collectively executed by the at least one processor, causes the electronic device to, based on identifying that a first wearable electronic device (e.g., the first wearable electronic device 201 of FIG. 2) and a second wearable electronic device (e.g., the second wearable electronic device 301 of FIG. 2) are communicatively connected to the electronic device by using the communication circuitry, control the display to display a first message for selecting a skin temperature measuring device at the start time point.

According to an embodiment, the memory may store an instruction that, when individually or collectively executed by the at least one processor, causes the electronic device to, based on identifying that the first wearable electronic device is selected as the skin temperature measuring device among the first wearable electronic device and the second wearable electronic device, transmit, to the first wearable electronic device, a first command to cause the first wearable electronic device to measure the skin temperature of the user from the start time point to the end time point.

According to an embodiment, the memory may store an instruction that, when individually or collectively executed by the at least one processor, causes the electronic device to, after transmitting the first command, control the display to display a second message inducing the user to keep wearing the first wearable electronic device during a first specified period of time.

According to an embodiment, the memory may store an instruction that, when individually or collectively executed by the at least one processor, causes the electronic device to predict the menstrual cycle of the user, based on skin temperature information of the user measured by the first wearable electronic device from the start time point to the end time point.

According to an embodiment, the instructions, when individually or collectively executed by the at least one processor, may cause the electronic device to, based on identifying that the second wearable electronic device is selected as the skin temperature measuring device among the first wearable electronic device and the second wearable electronic device, transmit, to the second wearable electronic device, a second command to cause the second wearable electronic device to measure the skin temperature of the user from the start time point to the end time point, control the display to display a third message inducing the user to keep wearing the second wearable electronic device during the first specified period of time, and predict the menstrual cycle of the user, based on skin temperature information of the user measured by the second wearable electronic device from the start time point to the end time point.

According to an embodiment, the first wearable electronic device may be configured in the form of a ring wearable on a first body part of the user, and the second wearable electronic device may be configured in the form of a watch wearable on a second body part of the user that is different from the first body part.

According to an embodiment, the instructions, when individually or collectively executed by the at least one processor, may cause the electronic device to control the display to display a fourth message inducing wearing of the first wearable electronic device, based on identifying that the selected first wearable electronic device is not worn during a second specified period of time after the start time point.

According to an embodiment, the instructions, when individually or collectively executed by the at least one processor, may cause the electronic device to control the display to display a first execution screen including the configuration information and the skin temperature information measured by the first wearable electronic device.

According to an embodiment, the configuration information may include menstrual cycle information configured based on user input information and/or based on skin temperature information measured by the first wearable electronic device before executing the function for predicting the menstrual cycle.

According to an embodiment, the instructions may store instructions that, when individually or collectively executed by the at least one processor, cause the electronic device to, based on identifying a request for changing the skin temperature measuring device before the end time point, control the display to display a fifth message indicating an importance of predicting the menstrual cycle and guiding the user to change the skin temperature measuring device at a start time point of a next cycle.

According to an embodiment, the instructions, when individually or collectively executed by the at least one processor, may cause the electronic device to, based on identifying that the selected first wearable electronic device is not worn during the specified period of time and the second wearable electronic device is worn, obtain second skin temperature information measured by the second wearable electronic device during the specified period of time, and correct the second skin temperature information by a skin temperature difference value.

According to an embodiment, the skin temperature difference value may be configured as a difference between first skin temperature values measured by the first wearable electronic device in a previous cycle and second skin temperature values measured by the second wearable electronic device in the previous cycle.

According to an embodiment, the instructions, when individually or collectively executed by the at least one processor, may cause the electronic device to, based on identifying a request for changing the skin temperature measuring device before a predicted fertile period end date, control the display to display a sixth message not recommending changing of the skin temperature measuring device.

According to an embodiment, the instructions, when individually or collectively executed by the at least one processor, may cause the electronic device to, after identifying the request for changing the skin temperature measuring device before the predicted fertile period end date, based on identifying that the skin temperature measuring device is not changed, control the display to display a seventh message for identifying whether to change the skin temperature measuring device on the predicted fertile period end date.

According to an embodiment, the instructions, when individually or collectively executed by the at least one processor, may cause the electronic device to control the display to display an object indicating a reservation for changing of the skin temperature measuring device on the first execution screen during a specified period of time before the start time point of the next cycle, based on identifying an input for selecting the object, control the display to display an eighth message guiding that the skin temperature measuring device is changed from the first wearable electronic device to the second wearable electronic device before the start time point of the next cycle, and control the display to display a ninth message recommending wearing of the second wearable electronic device at the start time point of the next cycle. In this way, by providing guidance functions such as reminders and insight messages at the start of the menstrual cycle, users can more effectively select or change their skin temperature measuring device, thereby enhancing the accuracy of menstrual cycle predictions.

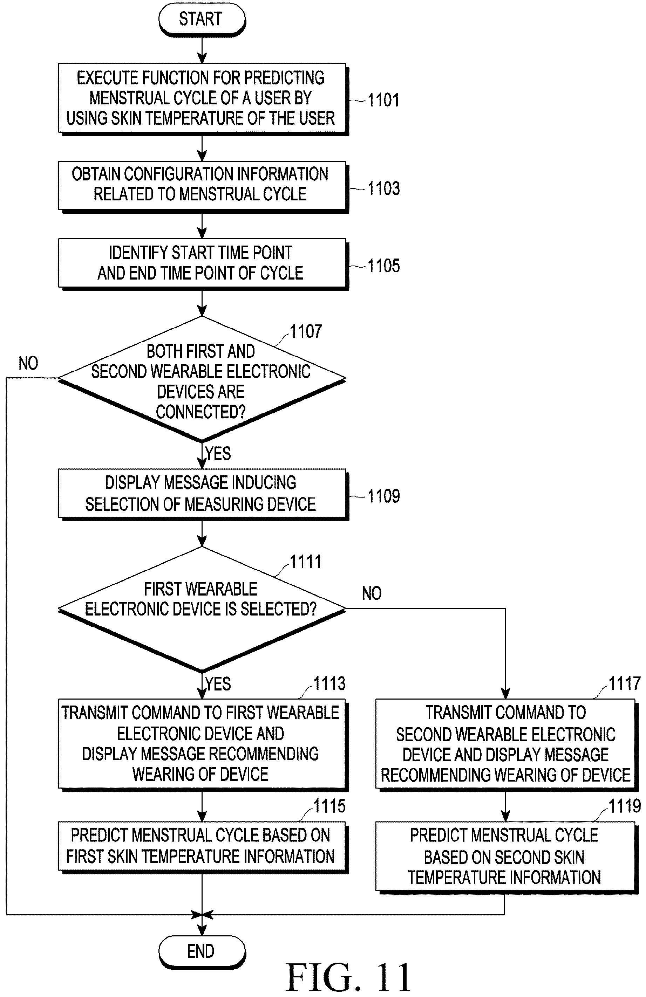

FIG. 11 illustrates an example of an operation method in an electronic device according to an embodiment. In the following embodiments, each operation may be performed sequentially, but is not necessarily performed sequentially. For example, the order of each operation may be changed, and at least two operations may be performed in parallel.

Referring to FIG. 11, in operation 1101, an electronic device (e.g., the electronic device 101 of FIGS. 1 and 2) according to an embodiment may identify an execution request of a function for predicting a menstrual cycle of a user by using skin temperature. The electronic device may execute a function (e.g., an application, a program, or an operation) for predicting a menstrual cycle by using skin temperature, and may display an execution screen (e.g., the first execution screen 310 or a main screen of FIG. 3A) according to the execution of the function on a display (e.g., the display module of FIG. 1 or the display 161 of FIGS. 3A to 5D and FIGS. 7A to 10).

In operation 1103, the electronic device may obtain configuration information related to the menstrual cycle of the user stored in memory (e.g., the memory 130 of FIG. 1), based on identifying an execution request of a function for menstrual cycle prediction. The configuration information may be pre-configured and stored in the memory based on user input information or information related to a menstrual cycle predicted in a previous cycle, before identifying the execution request of the function. The user input information is information directly input by the user by using an input interface of a first execution screen, and may include the user's gender, age, menstrual cycle, menstrual start date of a previous cycle, and/or menstrual end date of the previous cycle. The information related to the predicted menstrual cycle is information predicted (or updated) based on skin temperature information measured in the previous cycle, and may include at least one of predicted menstrual cycle, predicted menstrual start date, predicted menstrual end date, predicted ovulation date, and predicted fertile period information.

In operation 1105, the electronic device may identify a start time point and an end time point of a cycle for measuring skin temperature of the user, based on the configuration information. The cycle for measuring skin temperature may be configured, for example, from a predicted menstrual start date to a predicted fertile period end date, from the predicted menstrual start date to the day before the next predicted menstrual start date, or from the predicted menstrual start date to a specified date between the predicted fertile period end date and the day before the next predicted menstrual start date. The cycle for measuring skin temperature may be configured to be the same as or different from a menstrual cycle (e.g., 30 days) included in the configuration information.

In operation 1107, the electronic device may use communication circuitry to identify whether a connected wearable device is connected to the electronic device. As a result of the identification, if it is identified that two or more wearable electronic devices (e.g., the first wearable electronic device 201 of FIG. 2 and the second wearable electronic device 301 of FIG. 3)) are connected to the electronic device, the electronic device may perform operation 1109. As a result of the identification, when two or more wearable electronic devices are not connected, the electronic device may terminate the operation method of FIG. 11. For example, when two or more wearable electronic devices are not connected, the electronic device may perform a generally known function for predicting a menstrual cycle, without performing the operation method of FIG. 11 according to an embodiment, and thus a detailed description thereof will be omitted.

In operation 1109, the electronic device may display, on the display, a first message (e.g., the message 510 of FIG. 5A) for selecting a wearable electronic device for measuring skin temperature of the user at the identified start time point, based on identifying that two or more wearable electronic devices (hereinafter, referred to as a first wearable electronic device and a second wearable electronic device) are all connected. The first message may include content (e.g., the content 511) that guides selection of a wearable electronic device for measuring skin temperature, and function buttons (e.g., the watch selection button 513, the ring selection button 515, a cancel button, and/or an ok button). The electronic device may display the first message on a part of the first execution screen (e.g., the first execution screen 310 of FIG. 3A) displayed on the display or through a separate pop-up window adjacent to or overlapping the first execution screen 310.

In operation 1111, the electronic device may identify whether the first wearable electronic device, which is one of the first wearable electronic device and the second wearable electronic device, is selected. When the user selects one of the function buttons (e.g., the watch selection button 513 and the ring selection button 515 of FIG. 5A) included in the first message, the electronic device may identify the selection of the first wearable electronic device or the second wearable electronic device. As a result of the identification, if it is identified that the first wearable electronic device is selected, the electronic device may perform operation 1113, and if it is identified that the second wearable electronic device is selected, the electronic device may perform operation 1117.

In operation 1113, the electronic device may transmit, to the first wearable electronic device, a command to cause the first wearable electronic device to measure skin temperature of the user from the start time point to the end time point of the cycle, based on identifying that the first wearable electronic device is selected. After transmitting the command to the first wearable electronic device, the electronic device may control the display to display a second message (e.g., the message 520 of FIG. 5B) recommending (e.g., inducing or guiding) the user to keep wearing the first wearable electronic device during a specified period of time. The second message may include content (e.g., "Wear the ring while sleeping") that induces the user to wear the first wearable electronic device (e.g., a ring) at bedtime and content (e.g., "Please keep wearing the ring during bedtime") that induces the user to keep wearing the device. The electronic device may display the second message on a part of the first execution screen or through a separate pop-up window adjacent to or overlapping the first execution screen.

In operation 1115, the electronic device may predict the menstrual cycle of the user, based on skin temperature information (e.g., first skin temperature information) measured at a first body part of the user by the first wearable electronic device from the start time point to the end time point of the cycle. The electronic device may terminate the operation method after operation 1115.

In operation 1117, the electronic device may transmit, to the second wearable electronic device, a command to measure skin temperature of the user from the start time point to the end time point of the cycle, based on identifying that the second wearable electronic device is selected. After transmitting the command to the second wearable electronic device, the electronic device may control the display to display a third message (e.g., the message 730 of FIG. 7C) recommending (e.g., inducing or guiding) the user to keep wearing the second wearable electronic device during a specified period of time (e.g., bedtime) The third message may include content (e.g., "Wear the watch at bedtime") that induces the user to wear the second wearable electronic device (e.g., a watch) at bedtime and content (e.g., "Please keep wearing the watch during bedtime") that induces the user to keep wearing the device. The electronic device may display the third message on a part of the first execution screen or through a separate pop-up window adjacent to or overlapping the first execution screen.

In operation 1119, the electronic device may predict the menstrual cycle of the user, based on skin temperature information (e.g., second skin temperature information) measured at a second body part of the user by the second wearable electronic device from the start time point to the end time point of the cycle. The electronic device may terminate the operation method after operation 1119.

When performing the operations 1115 and 1119 described above, the electronic device according to an embodiment may update a menstrual cycle predicted in a previous cycle to a menstrual cycle predicted in a current cycle. The electronic device may obtain information related to the menstrual cycle predicted in the current cycle and display the obtained information related to the menstrual cycle on the first execution screen (e.g., reconfigure and display the first execution screen based on the obtained information related to the menstrual cycle).

In operation 1113 of FIG. 11 described above, when skin temperature is measured by the unselected second wearable electronic device or skin temperature information measured by the selected first wearable electronic device is not collected, the electronic device according to an embodiment may apply a message or a graphic effect indicating that there is no measured skin temperature information to the user interface 313, that displays measured skin temperature information, of the first execution screen (e.g., a main screen) and a second execution screen (e.g., a detailed page or detailed screen for menstrual cycle prediction), or apply a graphic effect to a corresponding area of a user interface that displays measured skin temperature information such that second skin temperature information measured by the unselected second wearable electronic device is not displayed or is hidden.

In operation 1117 of FIG. 11 described above, when skin temperature is measured by the unselected first wearable electronic device or skin temperature information measured by the selected second wearable electronic device is not collected, the electronic device according to an embodiment may apply a message or a graphic effect indicating that there is no measured skin temperature information to a user interface, that displays measured skin temperature information, of the first execution screen (e.g., a main screen) and the second execution screen (e.g., a detailed page or detailed screen for menstrual cycle prediction), or apply a graphic effect to a user interface that displays measured skin temperature information such that second skin temperature information measured by the unselected first wearable electronic device is not displayed (e.g., displayed as an empty area or blurred) or is hidden.

According to an embodiment, when performing operation 1113 or 1117 of FIG. 11, if a wearable electronic device (e.g., the first wearable electronic device) different from a wearable electronic device (e.g., the second wearable electronic device) previously selected by the user or selected by default is selected by the user, the electronic device may display a message (e.g., the message 530 of FIG. 5C) for identifying whether to change a skin temperature measuring device. For example, the processor 120 may not display the message for identifying whether to change the skin temperature measuring device, depending on a function configuration. The message for identifying whether to change the skin temperature measuring device may include guidance content on changing of the skin temperature measuring device (e.g., "Would you like to change the measuring device?" and "The skin temperature data previously recorded by the watch will be hidden. If you choose to switch back, the data will be viewable again.") and function buttons (e.g., a cancel button and an ok button). The electronic device may control the display 161 to display the message for identifying whether to change the skin temperature measuring device on a part of the first execution screen 310 or through a separate pop-up window adjacent to or overlapping the first execution screen 310. When the user selects a button (ok button) for accepting a change of the skin temperature measuring device, the electronic device may display skin temperature information (e.g., first skin temperature information) measured by the selected wearable electronic device (e.g., the first wearable electronic device 201) on the first execution screen (e.g., the third user interface 313 of FIG. 3A).

According to an embodiment, when performing operation 1115 or 1119 of FIG. 11, if it is identified that the selected wearable electronic device (e.g., the first wearable electronic device 201) is not worn during a specified period of time (e.g., 2 days) after the start time point of the cycle, the electronic device may control the display 161 to display a message (e.g., a fourth message, the message 520 of FIG. 5B) for inducing (reminding) wearing of the selected wearable electronic device, while displaying, on the first execution screen 310, a message (e.g., the message 540 of FIG. 5D) for identifying whether skin temperature information is not displayed and guiding that the skin temperature measuring device can be changed. The processor 120 may display the fourth message on the first execution screen 310 or through a separate pop-up window adjacent to or overlapping the first execution screen 310.

According to an embodiment, when performing operation 1115 or 1119 of FIG. 11, if it is identified that a request for changing the skin temperature measuring device before the end time point of the cycle for measuring skin temperature (e.g., just before a predicted fertile period or at the beginning of a menstrual cycle), which is a non-recommended time, the electronic device may control the display to display a fifth message (e.g., the message 710 of FIG. 7A) indicating the importance of menstrual cycle prediction and recommending changing of the skin temperature measuring device at a start time point of the next cycle. The electronic device may display the fifth message on a part of the first execution screen (e.g., the first execution screen 310 or a main screen of FIG. 3) or through a separate pop-up window adjacent to or overlapping the first execution screen. For example, the fifth message may include content indicating the importance of menstrual cycle prediction and recommending changing of the skin temperature measuring device at the start time point of the next cycle (e.g., "Would you like to change the skin temperature measuring device?" and "This is an important time for collecting data necessary for cycle prediction. To improve prediction accuracy, it is recommended to change the device between the end of a fertile period and the next predicted menstrual start date.") and function buttons (e.g., a cancel button and an ok button). For example, when the user inputs an acceptance button (ok button) included in the fifth message, the electronic device may configure the skin temperature measuring device to be changed, transmit a command to measure skin temperature during a specified period of time of the cycle to a wearable electronic device (e.g., the second wearable electronic device) changed for measuring skin temperature, and display the first execution screen (e.g., a main screen).

According to an embodiment, when the electronic device identifies a request for changing the skin temperature measuring device before a predicted fertile period end date, which is a non-recommended time, the electronic device may control the display 161 to display a message (e.g., a sixth message) not recommending changing of the skin temperature measuring device. For example, the electronic device may display a sixth message including content that does not recommend changing of the skin temperature measuring device (e.g., "Device change is not recommended. Measure skin temperature with the selected ring"). For example, the electronic device may display the fifth message indicating the importance of menstrual cycle prediction and guiding the user to change the skin temperature measuring device at the start time point of the next cycle, while also displaying the sixth message including the content that does not recommend changing of the skin temperature measuring device.

According to an embodiment, when performing operation 1115 or 1119 of FIG. 11, when it is the end time point of the cycle for measuring skin temperature (e.g., after a predicted fertile period end date or before a start time point of the next cycle), the electronic device may control the display to display a message (e.g., the message 720 of FIG. 7B) for identifying whether to change the skin temperature measuring device. For example, when the user inputs a button (e.g., a watch) indicating the second wearable electronic device (e.g., the second wearable electronic device 301 of FIG. 3) in the message for identifying whether to change the device, the electronic device may change the skin temperature measuring device from the first wearable electronic device to the second wearable electronic device, and transmit a command to measure skin temperature during a specified period of time of the cycle to the second wearable electronic device. According to an embodiment, the electronic device may display a message (e.g., the message 730 of FIG. 7C) inducing wearing of the second wearable electronic device at the start time point of the next cycle.

According to an embodiment, when performing operation 1115 or 1119 of FIG. 11, when it is the end time point of the cycle for measuring skin temperature (e.g., after a predicted fertile period end date), the electronic device may identify whether the user has attempted to change the skin temperature measuring device before the end time point. When the user has attempted to change the device before the end time point but keeps the selected wearable electronic device instead of changing the device, the electronic device may control the display to display a seventh message (e.g., the message 810 of FIG. 8) for identifying whether to change the skin temperature measuring device. The seventh message may include content for identifying whether to change the skin temperature measuring device (e.g., "Would you like to change the skin temperature measuring device?" and "The predicted fertile period has passed. Would you like to change the skin temperature measuring device?") and function buttons (e.g., a cancel button and an ok button). For example, when the user inputs an acceptance button (ok button) in the seventh message, the electronic device may change the skin temperature measuring device from the first wearable electronic device to the second wearable electronic device, transmit a command to measure skin temperature during a specified period of time of the cycle to the second wearable electronic device, and control the display to display the first execution screen (e.g., a main screen). According to an embodiment, when a predicted fertile period has not ended or there is no attempt to change the skin temperature measuring device just before the predicted fertile period, the electronic device may control the display to display the first execution screen (e.g., a main screen) without displaying a message (e.g., a seventh message) for identifying whether to change the skin temperature measuring device.

According to an embodiment, when performing operation 1115 or 1119 of FIG. 11, the electronic device may control the display to display a function button (e.g., a menu, an object, or a graphic element) (e.g., the function button 911 of FIG. 9) for reserving a change of the skin temperature measuring device before the start time point of the next menstrual cycle on the first execution screen (e.g., a main screen). The function button may be displayed in an area below a third user interface of the first execution screen and may be displayed during a specified period of time (e.g., from 5 days before a predicted menstrual end date to a menstrual cycle end date). When the user selects the function button, the electronic device may control the display to display a message (e.g., an eighth message, the message 913 of FIG. 9) guiding that the wearable electronic device for measuring skin temperature is changed to the second wearable electronic device (e.g., a watch) before the start time point of the next menstrual cycle. The electronic device may control the display to display a message (e.g., a ninth message, the message 915 of FIG. 9) inducing (e.g., recommending) wearing of the second wearable electronic device to measure skin temperature by using the changed second wearable electronic device at the start time point of the next cycle for measuring skin temperature.

According to an embodiment, when performing operation 1115 or 1119 of FIG. 11, the electronic device may identify that the selected first wearable electronic device (e.g., a ring) is not worn during a specified period of time and the unselected second wearable electronic device (e.g., a watch) is worn. If it is identified that the unselected second wearable electronic device is worn, the electronic device may collect second skin temperature information measured by the unselected second wearable electronic device during a specified period of time in the cycle. The electronic device may correct the second skin temperature information by a skin temperature difference value in order to predict an accurate menstrual cycle. The skin temperature difference value may be configured (e.g., specified or calculated) as the difference between first skin temperature values measured by the first wearable electronic device and second skin temperature values measured by the second wearable electronic device during a specified period of time in a previous cycle. The first wearable electronic device may be worn on a first body part (e.g., a finger) with a higher blood vessel distribution due to the body structure, and the second wearable electronic device is worn on a body part with a relatively fewer blood vessel distribution and having more skin exposed to an external environment compared to the first body part (e.g., a finger), and thus may be relatively more affected by an external environmental temperature. The first body part on which the first wearable electronic device is worn may be measured to have a higher skin temperature than that of a second body part (e.g., a wrist) on which the second wearable electronic device is worn. In this way, by providing guidance functions such as reminders and insight messages at the start of the menstrual cycle, users can more effectively select or change their skin temperature measuring device, thereby enhancing the accuracy of menstrual cycle predictions.

According to an embodiment, an operation method in an electronic device (e.g., the electronic device 101 of FIGS. 1 and 2) may include, executing a function for predicting a menstrual cycle of a user by using skin temperature of the user.

According to an embodiment, the method may include, based on identifying an execution request of the function for predicting the menstrual cycle of the user by using skin temperature, obtaining configuration information related to the menstrual cycle of the user stored in memory (e.g., the memory 130 of FIG. 1) of the electronic device.

According to an embodiment, the method may include identifying a start time point and an end time point of a cycle for measuring skin temperature of the user, based on the configuration information.

According to an embodiment, the method may include, based on identifying that a first wearable electronic device (e.g., the first wearable electronic device 201 of FIG. 2) and a second wearable electronic device (e.g., the second wearable electronic device 301 of FIG. 2) are communicatively connected to the electronic device by using communication circuitry (e.g., the communication module 190 of FIG. 1) of the electronic device, controlling a display (e.g., the display module 160 of FIG. 1 or the display 161 of FIGS. 3A to 5D and FIG. 9) of the electronic device to display a first message for selecting a skin temperature measuring device at the start time point.

According to an embodiment, the method may include, based on identifying that the first wearable electronic device is selected as the skin temperature measuring device among the first wearable electronic device and the second wearable electronic device, transmitting, to the first wearable electronic device, a first command to cause the first wearable electronic device to measure the skin temperature of the user from the start time point to the end time point.

According to an embodiment, the method may include, after transmitting the first command, controlling the display to display a second message inducing the user to keep wearing the first wearable electronic device during a first specified period of time.

According to an embodiment, the method may include predicting the menstrual cycle of the user, based on skin temperature information of the user measured by the first wearable electronic device from the start time point to the end time point.

According to an embodiment, the method may further include, based on identifying that the second wearable electronic device is selected as the skin temperature measuring device among the first wearable electronic device and the second wearable electronic device, transmitting, to the second wearable electronic device, a second command to cause the second wearable electronic device to measure the skin temperature of the user from the start time point to the end time point, controlling the display to display a third message inducing the user to keep wearing the second wearable electronic device during the first specified period of time, and predicting the menstrual cycle of the user, based on skin temperature information of the user measured by the second wearable electronic device from the start time point to the end time point.

According to an embodiment, the first wearable electronic device may be configured in a form of a ring wearable on a first body part of the user.

According to an embodiment, the second wearable electronic device may be configured in a form of a watch wearable on a second body part of the user that is different from the first body part.

According to an embodiment, the method may further include controlling the display to display a fourth message inducing wearing of the first wearable electronic device, based on identifying that the selected first wearable electronic device is not worn during a second specified period of time after the start time point.

According to an embodiment, the method may further include controlling the display to display a first execution screen including the configuration information and the skin temperature information measured by the first wearable electronic device.

The configuration information may include menstrual cycle information configured based on user input information and/or based on skin temperature information measured by the first wearable electronic device before executing the function for predicting the menstrual cycle.

According to an embodiment, the predicting of the menstrual cycle of the user may include, based on identifying a request for changing the skin temperature measuring device before the end time point, controlling the display to display a fifth message indicating an importance of predicting the menstrual cycle and guiding the user to change the skin temperature measuring device at a start time point of a next cycle.

According to an embodiment, the predicting of the menstrual cycle of the user may include, based on identifying that the selected first wearable electronic device is not worn during the first specified period of time and the second wearable electronic device is worn, obtaining second skin temperature information measured by the second wearable electronic device during the first specified period of time, and correcting the second skin temperature information by a skin temperature difference value.

According to an embodiment, the skin temperature difference value may be configured as a difference between first skin temperature values measured by the first wearable electronic device in a previous cycle and second skin temperature values measured by the second wearable electronic device in the previous cycle.

According to an embodiment, the predicting of the menstrual cycle of the user may include, based on identifying a request for changing the skin temperature measuring device before a predicted fertile period end date, controlling the display to display a sixth message not recommending changing of the skin temperature measuring device.

According to an embodiment, the method may include, after identifying the request for changing the skin temperature measuring device before the predicted fertile period end date, based on identifying that the skin temperature measuring device is not changed, displaying a seventh message for identifying whether to change the skin temperature measuring device on the predicted fertile period end date.

According to an embodiment, the method may further include controlling the display to display an object indicating a reservation for changing of the skin temperature measuring device on the first execution screen during a specified period of time before the start time point of the next cycle, based on identifying an input for selecting the object, controlling the display to display an eighth message guiding that the skin temperature measuring device is changed from the first wearable electronic device to the second wearable electronic device before the start time point of the next cycle, and controlling the display to display a ninth message recommending wearing of the second wearable electronic device at the start time point of the next cycle.

According to an embodiment, in a non-transitory storage medium storing one or more programs, the programs may include instructions that, when executed by at least one processor (e.g., the processor 120 of FIG. 1) of an electronic device (e.g., the electronic device 101 of FIGS. 1 and 2), cause the electronic device to execute, based on identifying an execution request of a function for predicting a menstrual cycle of a user by using skin temperature, obtaining configuration information related to the menstrual cycle of the user stored in memory (e.g., the memory 130 of FIG. 1) of the electronic device, identifying a start time point and an end time point of a cycle for measuring skin temperature of the user, based on the configuration information, based on identifying that a first wearable electronic device (e.g., the first wearable electronic device 201 of FIG. 2) and a second wearable electronic device (e.g., the second wearable electronic device 301 of FIG. 2) are connected to the electronic device by using communication circuitry (e.g., the communication module 190 of FIG. 1) of the electronic device, controlling a display (e.g., the display 160 of FIG. 1 or the display(161) of FIGS. 3A to 4B, 5C, 5D and FIG. 9) of the electronic device to display a first message for selecting a wearable electronic device for measuring the skin temperature of the user at the start time point, based on identifying that the first wearable electronic device is selected among the first wearable electronic device 201 and the second wearable electronic device, transmitting, to the first wearable electronic device, a command to cause the first wearable electronic device to measure the skin temperature of the user from the start time point to the end time point, after transmitting the command, controlling the display to display a second message inducing the user to keep wearing the first wearable electronic device during a first specified period of time, and predicting the menstrual cycle of the user, based on skin temperature information of the user measured by the first wearable electronic device from the start time point to the end time point.

In the disclosure, when an electronic device is connected to a plurality of wearable electronic devices and executes a function for predicting a menstrual cycle, the electronic device selects one of the plurality of wearable electronic devices to measure skin temperature used for predicting the menstrual cycle, and predicts the menstrual cycle based on skin temperature information measured by the selected wearable electronic device, thereby predicting a more accurate menstrual cycle, and provides a message related to selection or change of the wearable electronic devices, thereby facilitating selection or change of a skin temperature measuring device. In addition to this, various effects identified directly or indirectly through this document may be provided. The effects obtainable from the disclosure are not limited to the above-mentioned effects, and other effects not mentioned may be clearly understood by a person skilled in the art to which the disclosure belongs from the following description.

Further, the embodiments disclosed herein are provided merely to describe technical contents and to help the understanding of the technical contents, and are not intended to limit the scope of the technology described herein. Therefore, it should be construed that the scope of the disclosure includes any change or other various embodiments based on the technical idea of this document.

The electronic device according to an embodiment may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that an embodiment of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with an embodiment of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

An embodiment as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to an embodiment of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to an embodiment, operations performed by the module, the program, or another component may be carried out sequentially, in parallel, repeatedly, or heuristically, or one or more of the operations may be executed in a different order or omitted, or one or more other operations may be added.

## Claims

1. An electronic device (101) comprising:
a display (160, 161);
communication circuitry (190);
at least one processor (120); and
memory (130) storing instructions that, when executed by the at least one processor individually or collectively, cause the electronic device to:
execute a function for predicting a menstrual cycle of a user by using skin temperature of the user;
based on identifying an execution request of the function for predicting the menstrual cycle of the user using skin temperature of the user, obtain configuration information related to the menstrual cycle of the user stored in the memory;
identify a start time point and an end time point of a cycle for measuring the skin temperature of the user based on the configuration information;
based on identifying that a first wearable electronic device (201) and a second wearable electronic device (301) are communicatively connected to the electronic device using the communication circuitry, control the display to display a first message for selecting a skin temperature measuring device at the start time point;
based on identifying that the first wearable electronic device is selected as the skin temperature measuring device among the first wearable electronic device and the second wearable electronic device, transmit a first command to the first wearable electronic device to measure the skin temperature of the user from the start time point to the end time point;
after transmitting the first command, control the display to display a second message inducing the user to keep wearing the first wearable electronic device during a first specified period of time; and
predict the menstrual cycle of the user based on skin temperature information of the user measured by the first wearable electronic device from the start time point to the end time point.

2. The electronic device of claim 1, wherein the instructions, when executed by the at least one processor individually or collectively, cause the electronic device to:
based on identifying that the second wearable electronic device is selected as the skin temperature measuring device among the first wearable electronic device and the second wearable electronic device, transmit a second command to the second wearable electronic device to measure the skin temperature of the user from the start time point to the end time point;
control the display to display a third message recommending the user to keep wearing the second wearable electronic device during the first specified period of time; and
predict the menstrual cycle of the user based on skin temperature information of the user measured by the second wearable electronic device from the start time point to the end time point,
wherein the first wearable electronic device is configured in a form of a ring wearable on a first body part of the user, and
wherein the second wearable electronic device is configured in a form of a watch wearable on a second body part of the user that is different from the first body part.

3. The electronic device of claim 1, wherein the instructions, when executed by the at least one processor individually or collectively, cause the electronic device to:
based on identifying that the selected first wearable electronic device has not been worn during a second specified period of time after the start time point, control the display to display a fourth message inducing wearing of the first wearable electronic device; and
control the display to display a first execution screen including the configuration information and the skin temperature information measured by the first wearable electronic device, and
wherein the configuration information is set based on user input information and/or based on skin temperature information measured by the first wearable device before executing the function for predicting the menstrual cycle.

4. The electronic device of any one of claims 1 to 3, wherein the instructions, when executed by the at least one processor individually or collectively, cause the electronic device to:
based on identifying a request for changing the skin temperature measuring device prior to the end time point, control the display to display a fifth message indicating an importance of predicting the menstrual cycle and guiding to change the skin temperature measuring device at a start time point of a next cycle.

5. The electronic device of any one of claims 1 to 4, wherein the instructions, when executed by the at least one processor individually or collectively, cause the electronic device to:
based on identifying that the selected first wearable electronic device is not worn during the specified period of time and the second wearable electronic device is worn,
obtain second skin temperature information measured by the second wearable electronic device during the specified period of time, and
correct the second skin temperature information by a skin temperature difference value,
wherein the skin temperature difference value is set as a difference between first skin temperature values measured by the first wearable electronic device in a previous cycle and second skin temperature values measured by the second wearable electronic device in the previous cycle.

6. The electronic device of any one of claims 1 to 5, wherein the instructions, when executed by the at least one processor individually or collectively, cause the electronic device to:
based on identifying a request for changing the skin temperature measuring device before an end day of a predicted fertility period, control the display to display a sixth message not recommending changing of the skin temperature measuring device; and
after identifying the request for changing the skin temperature measuring device before the end day of the predicted fertility period, based on identifying that the wearable electronic device for measuring the skin temperature has not been changed, control the display to display a seventh message for determining whether to change the skin temperature measuring device on the end day of the predicted fertility period.

7. The electronic device of any one of claims 1 to 6, wherein the instructions, when executed by the at least one processor individually or collectively, cause the electronic device to:
control the display to display an object indicating a reservation for changing of the skin temperature measuring device on the first execution screen during a specified period of time before the start time point of the next cycle;
based on identifying an input for selecting the object, control the display to display an eighth message guiding that the skin temperature measuring device is changed from the first wearable electronic device to the second wearable electronic device before the start time point of the next cycle; and
control the display to display a ninth message inducing wearing of the second wearable electronic device at the start time point of the next cycle.

8. An operation method of an electronic device (101), the method comprising:
executing a function for predicting a menstrual cycle of a user by using skin temperature of the user;
based on identifying an execution request of the function for predicting the menstrual cycle of the user by using skin temperature, obtaining configuration information related to the menstrual cycle of the user stored in memory (130) of the electronic device;
identifying a start time point and an end time point of a cycle for measuring skin temperature of the user, based on the configuration information;
based on identifying that a first wearable electronic device (201) and a second wearable electronic device (301) are communicatively connected to the electronic device by using communication circuitry (190) of the electronic device, controlling a display (160, 161) of the electronic device to display a first message for selecting a skin temperature measuring device at the start time point;
based on identifying that the first wearable electronic device is selected as the skin temperature measuring device among the first wearable electronic device (201) and the second wearable electronic device (301), transmitting, to the first wearable electronic device, a first command to cause the first wearable electronic device to measure the skin temperature of the user from the start time point to the end time point;
after transmitting the first command, controlling the display to display a second message inducing the user to keep wearing the first wearable electronic device during a first specified period of time; and
predicting the menstrual cycle of the user, based on skin temperature information of the user measured by the first wearable electronic device from the start time point to the end time point.

9. The method of claim 8, further comprising:
based on identifying that the second wearable electronic device is selected as the skin temperature measuring device among the first wearable electronic device (201) and the second wearable electronic device (301), transmitting, to the second wearable electronic device, a second command to cause the second wearable electronic device to measure the skin temperature of the user from the start time point to the end time point;
controlling the display to display a third message inducing the user to keep wearing the second wearable electronic device during the first specified period of time; and
predicting the menstrual cycle of the user, based on skin temperature information of the user measured by the second wearable electronic device from the start time point to the end time point,
wherein the first wearable electronic device is configured in a form of a ring wearable on a first body part of the user, and
wherein the second wearable electronic device is configured in a form of a watch wearable on a second body part of the user that is different from the first body part.

10. The method of one of claim 8 or 9, further comprising controlling the display to display a fourth message inducing wearing of the first wearable electronic device, based on identifying that the selected first wearable electronic device is not worn during a second specified period of time after the start time point; and
controlling the display to display a first execution screen comprising the configuration information and the skin temperature information measured by the first wearable electronic device,
wherein the configuration information comprises menstrual cycle information configured based on user input information and/or based on skin temperature information measured by the first wearable electronic device before executing the function for predicting the menstrual cycle.

11. The method of one of claims 8 to 10, wherein the predicting of the menstrual cycle of the user comprises, based on identifying a request for changing the skin temperature measuring device before the end time point, controlling the display to display a fifth message indicating an importance of predicting the menstrual cycle and guiding the user to change the skin temperature measuring device at a start time point of a next cycle.

12. The method of one of claims 8 to 11, wherein the predicting of the menstrual cycle of the user comprises:
based on identifying that the selected first wearable electronic device is not worn during the first specified period of time and the second wearable electronic device is worn, obtaining second skin temperature information measured by the second wearable electronic device during the first specified period of time; and
correcting the second skin temperature information by a skin temperature difference value,
wherein the skin temperature difference value is configured as a difference between first skin temperature values measured by the first wearable electronic device in a previous cycle and second skin temperature values measured by the second wearable electronic device in the previous cycle.

13. The method of one of claims 8 to 12, wherein the predicting of the menstrual cycle of the user comprises:
based on identifying a request for changing the skin temperature measuring device before a predicted fertile period end date, controlling the display to display a sixth message not recommending changing of the skin temperature measuring device; and
after identifying the request for changing the skin temperature measuring device before the predicted fertile period end date, based on identifying that the skin temperature measuring device is not changed, displaying a seventh message for identifying whether to change the skin temperature measuring device on the predicted fertile period end date.

14. The method of one of claims 8 to 13, further comprising:
controlling the display to display an object indicating a reservation for changing of the skin temperature measuring device on the first execution screen during a specified period of time before the start time point of the next cycle;
based on identifying an input for selecting the object, controlling the display to display an eighth message guiding that the skin temperature measuring device is changed from the first wearable electronic device to the second wearable electronic device before the start time point of the next cycle; and
controlling the display to display a ninth message recommending wearing of the second wearable electronic device at the start time point of the next cycle.

15. A non-transitory storage medium storing one or more programs, wherein the programs comprise instructions that, when executed by at least one processor (120) of an electronic device (101), cause the electronic device to execute:
execute a function for predicting a menstrual cycle of a user by using skin temperature of the user;
based on identifying an execution request of the function for predicting the menstrual cycle of the user by using skin temperature, obtaining configuration information related to the menstrual cycle of the user stored in memory (130) of the electronic device;
identify a start time point and an end time point of a cycle for measuring skin temperature of the user, based on the configuration information;
based on identifying that a first wearable electronic device (201) and a second wearable electronic device (301) are communicatively connected to the electronic device by using communication circuitry (190) of the electronic device, controlling a display (160, 161) of the electronic device to display a first message for selecting a skin temperature measuring device at the start time point;
based on identifying that the first wearable electronic device is selected as the skin temperature measuring device among the first wearable electronic device (201) and the second wearable electronic device (301), transmitting, to the first wearable electronic device, a first command to cause the first wearable electronic device to measure the skin temperature of the user from the start time point to the end time point;
after transmitting the first command, controlling the display to display a second message inducing the user to keep wearing the first wearable electronic device during a first specified period of time; and
predict the menstrual cycle of the user, based on skin temperature information of the user measured by the first wearable electronic device from the start time point to the end time point.
